# EUROPEAN PATENT APPLICATION

(11) **EP 0 904 787 A1**
(43) Date of publication of application: **31.03.1999**
(21) Application number: 97908497.7
(22) Date of filing: 19.03.1997
(51) Int. Cl.: A61K 47/30

(54) **SCLERAL PLUG CONTAINING PROTEINACEOUS MEDICINE**

(30) Priority: 25.03.1996 JP 68609/96
(71) Applicant: SANTEN PHARMACEUTICAL CO., LTD., Higashiyodogawa-ku, Osaka-shi, Osaka 533-8651 (JP)
(72) Inventor: OGURA, Yuichiro, Kyoto-shi, Kyoto 606 (JP); KUNOU, Noriyuki, Yawata-shi, Kyoto 614 (JP); OTA, Atsutoshi, Osaka-shi, Osaka 536 (JP)
(74) Representative: Barske, Heiko, Dr. rer. nat.
(86) International application number: JP9700939
(87) International publication number: WO9735621

(57) **Abstract**

The present invention provides a technique enabling a drug of protein derivatives such as an interferon-β to be effectively applied to intraocular diseases such as diseases of a vitreous body, retina, and the like. Namely, the present invention provides a composition comprising a drug of protein derivatives adsorbed on a surface of fine particles of a lactic acid copolymer, and also a scleral plug formed from the composition. The average diameter of the fine particles is less than 1,000 nanometers, preferably within a range of 50 to 500 nanometers. The drug of protein derivatives is, for example, an interferon, particularly an interferon-β. The lactic acid copolymer preferably comprises lactic acid units and glycolic acid units.

## Description

### Technical Field

The present invention provides a novel sustained-release composition of a drug of protein derivatives that is liable to be inactivated by heating or contact with an organic solvent. In particular, the present invention provides a technique of forming the composition into a scleral plug for allowing the drug to be released into a vitreous body for treatment or prevention of intraocular diseases.

### Background Techniques

Intraocular diseases such as diseases of a retina or vitreous body are often intractable, and a development of an effective treatment method is eagerly desired. Though ocular diseases are most generally treated by instillation of drugs, the drugs are hardly delivered to the intraocular tissues such as a retina and vitreous body, rendering the treatment of the intraocular diseases all the more difficult. An attempt was made to treat the diseases by intravenous administration or the like. However, because of a blood-aqueous barrier, it is difficult to allow the drug to be delivered to attain an effective concentration. A method is known in which the drug is directly injected into the vitreous body. However, injection of a high concentration drug all at once causes damage to intraocular tissues and, moreover, it is not practical to repeat the injection because of the danger of infection and the cumbersomeness of treatment procedure.

In view of this, a scleral plug made of a biodegradable copolymer was devised (See Japanese Laid-open Patent Publication No. HEI 06-312943). This scleral plug is formed from a lactic acid copolymer made of lactic acid units and glycolic acid units, containing a drug, whereby the drug is gradually released into a vitreous body by utilizing the biodegradability of the copolymer in order to treat the diseases in the vitreous body or retina. In addition, the scleral plug can be easily inserted into a small incision of sclera that is formed at the time of a vitreoretinal surgery or the like. The present invention has been made by applying a fundamental technique of scleral plug disclosed in Japanese Laid-open Patent Publication No. HEI 06-312943 and by adding thereto a novel technique for more effective application to a drug of protein derivatives that is liable to lose activity by heating or contact with an organic solvent.

In the meantime, numerous techniques using a microsphere were reported aiming at controlled release of drugs. Recently, there was reported a technique that utilizes nanospheres, i.e. fine particles having a particle diameter in the order of nanometers (See "Journal of Controlled Release, 25, 89-98, Japanese Laid-open Patent Publication No. HEI 05-58882, and the like). These techniques involve incorporating a drug into finely granulated capsules in order to control release of the drug. Further, the above-mentioned reports on nanospheres disclose nanospheres containing a copolymer of lactic acid and glycolic acid. To be more specific, they disclose a technique of incorporating a drug into capsules by dissolving the copolymer together with the drug in a water-miscible organic solvent, emulsifying the mixture finally, and evaporating the solvent. Since the copolymer is highly lipo-soluble, an organic solvent is generally used to dissolve the copolymer. Water-miscible organic solvents are used in the above-mentioned reports as well. Accordingly, it is difficult to use these techniques for drugs of protein derivatives that are liable to lose their activity by contact with an organic solvent.

On the other hand, a lot of known drugs of protein derivatives such as an interferon-β are expected to be effective on intraocular diseases such as diseases of a vitreous body or retina. In particular, the interferon-β exhibits neovascularization inhibiting property (See "Journal of Japanese Ophthalmic Society", 99, 571, 1995) and is expected to be effective on macular degeneration, diabetic retinopathy, central retinal vein obstruction, glaucoma caused by neovascularization, and the like. Further, the interferon-β also has an antiviral property, so that it is expected to be effective on various viral infectious diseases of intraocular tissues as well.

Although it is eagerly desired to develop a technique for applying drugs of protein derivatives such as an interferon-β effectively on intraocular diseases such as diseases of a vitreous body or retina, there is a fundamental problem that the drugs are hardly delivered to intraocular tissues such as a vitreous body or retina, and also there is a stability concern of the drugs of protein derivatives, i.e. a problem that the drugs of protein derivatives are liable to be inactivated by heating or contact with an organic solvent, so that the application has much technical difficulty.

### Disclosure of the Invention

The inventors paid their attention to a scleral plug made of a biodegradable lactic acid copolymer capable of releasing a drug easily and effectively into intraocular tissues such as a vitreous body and retina, and tried to incorporate a drug of protein derivatives such as interferon-β into the scleral plug. However, since the lactic acid copolymer is highly lipo-soluble, an organic solvent is necessary for homogeneously mixing the lactic acid copolymer with the drug. When the method was really applied to the drug of protein derivatives, the activity of the drug of protein derivatives was greatly lost. Further, although a known technique (Japanese Laid-open Patent Publication No. HEI 06-312943) discloses a method of forming a scleral plug by heat-molding as a specific example, this method can hardly be applied to the drugs of protein derivatives that are liable to be deactivated by heating.

Next, an attempt was made to incorporate the drug of protein derivatives into fine particles made of a lactic acid copolymer and forming the fine particles into a scleral plug by compression molding. However, owing to the high liposolubility of the lactic acid copolymer, an organic solvent is needed to incorporate the drug of protein derivatives into the fine particles, so that this technique was found to be unapplicable to drugs of protein derivatives.

Therefore, an intensive research was made to find a method of incorporating a drug of protein derivatives into a lactic acid copolymer substantially without contact with an organic solvent. Conventionally, the technique of using fine particles such as nanospheres aimed at incorporating the drug into the particles. This conventional concept was changed by noting the fact that proteins have an amino residue at their terminal and, hence, are easily bonded with a carboxylic residue of lactic acid copolymers. Thus, the inventors have found out that the drug can be homogeneously dispersed by allowing the drug to be adsorbed on a surface of the fine particles to utilize the extremely large surface area of the fine particles. The inventors also have found out that, according to this method, the drug of protein derivatives need not coexist in the steps of preparing the fine particles made of a lactic acid copolymer, so that there is substantially no contact of the drug of protein derivatives with an organic solvent and the activity of the drug does not decrease. In addition, the inventors have found out that the fine particles made of a lactic acid copolymer and having a drug of protein derivatives adsorbed on their surface can be easily formed into a scleral plug by means of the compression molding technique which does not need heating. Further, it has been found out that fine particles with their diameters in the order of nanometers can be easily formed into a scleral plug which is excellently homogeneous.

The present invention relates to a composition characterized by containing a drug of protein derivatives adsorbed on a surface of fine particles made of a lactic acid copolymer and a scleral plug formed from the composition.

In the present invention, the drugs of protein derivatives refer to pharmaceutical products containing protein in their composition, especially those that can be expected to have effects on intraocular diseases. Examples of the drugs of protein derivatives are interferons such as an interferon-β, various monoclonal antibodies, cytokines such as interleukins, anti-cytokine antibodies, and the like. These proteins can contain sugar chains.

The lactic acid copolymers refer to copolymers made of lactic acid units and glycolic acid units.

The fine particles refer to those having an extremely small average particle diameter, preferably those that are so-called "nanospheres" having an average particle diameter of less than 1,000 nanometers, more preferably those having an average particle diameter within a range of 50 to 500 nanometers.

The adsorption refers to a state in which the drug is substantially uniformly distributed on a surface of the particles and, physically, the drug is hardly desorbed from the surface of the particles.

The scleral plug refers to a biodegradable plug having shapes and properties disclosed in Japanese Laid-open Patent Publication No. HEI 06-312943.

Hereinbelow, a detailed explanation will be given.

According to the first aspect of the present invention, there is provided a composition characterized by containing a drug of protein derivatives adsorbed on a surface of fine particles made of a lactic acid copolymer. The composition is usually formed into a scleral plug as a final product. The composition can be also used as it is as a pharmaceutical product. A process for preparing the composition will be described briefly hereafter. First, a lactic acid copolymer is dissolved in an organic solvent. The obtained solution is added to an aqueous solution of polyvinyl alcohol while stirring, the mixture is filtrated. The filtrate is ultracentrifuged and the obtained precipitate is re-dispersed in water. Then the dispersion is ultracentrifuged again to give fine particles, followed by lyophilizing them to obtain fine particle powder. The fine particle powder is redispersed in water and an aqueous solution of a drug of protein derivatives is added thereto. The mixture is stirred and lyophilized to obtain the objective composition. According to this process, there is substantially no contact of the drug of protein derivatives with organic solvent, so that the activity of the drug of protein derivatives does not decrease.

According to the second aspect of the present invention, there is provided a scleral plug formed from the composition of the first aspect of the present invention. The scleral plug is mainly intended to be inserted through a small incision of a sclera formed at the time of vitreoretinal surgery to allow the drug to be gradually released into the vitreous body for treatment of intraocular diseases such as diseases of a retina or vitreous body. The shape and the strength of the scleral plug are determined according to known scleral plugs (See Japanese Laid-open Patent Publication No. HEI 6-312943). Also, since the present invention aims at sustained release of the drug, the molecular weight of the lactic acid copolymer and the compositional molar ratio of the units are determined according to the period of time required for the treatment (which varies depending on the kind of diseases, symptom, the age of the patient, etc.) or the like. For this purpose, the technique disclosed in Japanese Laid-open Patent Publication No. HEI 06-312943 can be directly applied. A brief description of this technique is given below.

First, the weight average molecular weight of the lactic acid copolymer is preferably within a range of 10,000 to 1,000,000. If the molecular weight is less than 10,000, the obtained scleral plug cannot have a sufficient strength. On the other hand, if the molecular weight exceeds 1,000,000, the degradation speed of the obtained plug will be too slow and also it will be difficult to mold the copolymer into the plug. The period of time for release of the drug (the period of time for maintaining effective concentration) is determined by appropriately selecting the molecular weight of the copolymer and the compositional molar ratio of the units. From a view point of molecular weight, a relationship between the period of time for release and the weight average molecular weight of the copolymer is shown below. For a period of time of about 1 to 2 weeks, the molecular weight is within a range of about 10,000 to 100,000, preferably about 10,000 to 50,000, more preferably about 20,000 to 40,000; for the period of about 2 weeks to 1 month, the molecular weight is about 10,000 to 200,000, preferably about 20,000 to 100,000, more preferably about 20,000 to 50,000; and for the period of about 1 month to 6 months, the molecular weight is about 10,000 to 1,000,000, preferably about 20,000 to 400,000, more preferably about 40,000 to 200,000. If the drug of protein derivatives is an interferon-β, the period of time of about 1 month to 6 months is preferable.

The compositional molar ratio of lactic acid units to glycolic acid units in the lactic acid copolymer also affects the degradability of the scleral plug. Namely, according as the compositional molar ratio of glycolic acid units increases, the hydrophilicity also increases, so that the water absorption will be higher to increase the degradation speed. If the compositional molar ratio of glycolic acid units is more than 50%, it will be difficult to form a scleral plug because of the solubility problem. Accordingly, the compositional molar ratio of lactic acid units to glycolic acid units in the copolymer is selected within a range of 50/50 to 100/0. If the drug is an interferon-β, the compositional molar ratio of lactic acid units to glycolic acid units in the copolymer is preferably within a range of 50/50 to 80/20, more preferably within a range of 70/30 to 80/20. The lactic acid units can be in L-, D-, or DL-form.

The lactic acid copolymer can be prepared by a known method, for example, by the process disclosed in Japanese Laid-open Patent Publication No. HEI 06-312943. Through the process, lactic acid copolymers of varying molecular weight and varying compositional molar ratio can be prepared.

The obtained copolymer can be formed into a scleral plug by utilizing the compression molding technique.

The scleral plug can have a shape equivalent to those disclosed in Japanese Laid-open Patent Publication No. HEI 06-312943. Briefly described, the scleral plug preferably has a nail-like shape that comprises a head portion that prevents the scleral plug from falling into the eye and a shaft portion to be inserted into a scleral incision. It is also preferable that the tip portion of the shaft has an acute-angled shape such as a cone or a pyramid. The scleral plug usually has a length of about 6 mm with the head portion having a diameter of about 2 mm and the shaft portion having a diameter or width of about 1 mm. The total weight of the scleral plug is about 9 mg.

The scleral plug of the present invention is mainly used for treatment or prevention of intraocular diseases such as diseases of a vitreous body of retina. However, the scleral plug can be applied to various diseases by selecting the kind of drug. For example, since the interferon-β has a neovascularization inhibiting property, it can be suitably applied to macular degeneration, diabetic retinopathy, central retinal vein obstruction, glaucoma caused by vascularization, and the like. Further, since the interferon-β also has an antiviral property, it can be applied to various viral infectious diseases of intraocular tissues as well.

### Best Modes for Carrying Out the Invention

### Example 1 (Preparation of fine particles of lactic acid copolymer)

A lactic acid copolymer (PLGA, 500 mg) having a weight average molecular weight of about 33,000 with a compositional molar ratio of DL-lactic acid/glycolic acid being 75/25 was dissolved in a mixture of methylene chloride (2 ml) and acetone (100 ml). The solution was added dropwise to an aqueous solution (2 w/v%, 200 ml) of polyvinyl alcohol (PVA) while stirring with a magnetic stirrer. After the mixture was stirred for two hours in vacuum by suction with an aspirator, the resulting suspension was filtrated with a membrane filter (hole diameter: 1µm). The filtrate was ultracentrifuged (156,000 × g) for one hour. The obtained precipitate was re-dispersed in purified water (q.s.) and then the dispersion was ultracentrifuged again to give fine particles. The fine particles were re-dispersed in purified water (15 ml) and then lyophilized to give 495 mg of fine particle powder.

The fine particles had an average particle diameter of about 100 nanometers, which confirmed that the particle diameter was in the order of nanometers.

In a similar manner as above, fine particles having a different particle diameter can be prepared by varying the mixing ratio of methylene chloride and acetone which were used as a solvent.

### Example 2 (Preparation of fine particles with a drug adsorbed on their surface)

The PLGA fine particle powder (125 mg) obtained in Example 1 was re-dispersed in purified water (2.5 ml). A solution (75 µl) of interferon-β (IFN-β) (7.5 × 10⁴ units, dissolved in a phosphoric acid buffer containing 0.1% of bovine serum albumin) and purified water (175 µ l) were added dropwise thereto. The mixture was stirred for 30 minutes with a magnetic stirrer, followed by lyophilization to give the objective fine particles with the drug adsorbed on their surface (IFN-β /PLGA powder).

### Example 3 (Formation of a scleral plug)

The fine particles with the drug adsorbed on their surface obtained in Example 2 were put into a mold for compression molding to form a scleral plug.

### Comparative Example 1 (Acetic acid dissolution method)

PLGA (125 mg, identical to the one used as a starting material in Example 1) was dissolved in acetic acid (2.5 ml). To the solution were dropwise added 75 µl of an IFN-β solution (identical to the one in Example 2) and purified water (175 µ l). Immediately after dissolution, the mixture was lyophilized to give IFN-β/PLGA powder.

### Comparative Example 2 (w/o type emulsion method)

PLGA (125 mg, identical to the one used as a starting material in Example 1) was dissolved in chloroform (2.5 ml). To the solution were dropwise added 75 µl of an IFN-β solution (identical to the one in Example 2) and purified water (175 µ l). After the dropwise addition was completed, a stable w/o type emulsion was prepared by ultrasonication (600w, 60 seconds) followed by lyophilization to give IFN-β/PLGA powder.

The present invention provides the following effects.

In order to verify whether or not fine particles of a lactic acid copolymer having a drug of protein derivatives adsorbed on their surface were obtained without decreasing the activity of the drug, a residual activity of the drug was measured. The activity of INF-β in the IFN-β /PLGA powder of Example 2 was measured, in which the interferon-β (IFN-β) was used as a representative example of the drug of protein derivatives. As comparative tests, the activity of INF-β in the IFN-β/PLGA obtained in Comparative Examples 1 and 2 was measured.

The activity was measured as follows. The IFN-β/PLGA powder (10 mg) was added to a mixture of purified water (2 ml) and chloroform (4 ml). After shaking for 30 minutes, the mixture was centrifuged (10 minutes), followed by measuring the activity of the IFN-β in the aqueous phase by means of ELISA. In this extraction operation, it was separately confirmed that the activity of the IFN-β did not decrease. The results are shown in the following Table.

The numerals in the Table show specific activities (average values of three examples) when the activity of the first IFN-β is assumed to be 100.

**TABLE 1**

| IFN-β/PLGA powder | Specific activity |
|---|---|
| Example 2 | 95.4 |
| Comparative Example 1 | 31.2 |
| Comparative Example 2 | 9.9 |

As is apparent from the results shown in this Table, little decrease of the IFN-β activity was observed in the IFN-β/PLGA powder of the present invention, whereas extremely large decrease of the IFN-β activity was observed in the solution method of Comparative Example 1 and the emulsion method of Comparative Example 2, in which the IFN-β was brought into contact with an organic solvent respectively.

### Industrial Applicability

As shown above, the present invention provides a novel sustained-release composition of a drug of protein derivatives that is liable to be inactivated by heating or contact with an organic solvent. In particular, the present invention provides a technique of forming the composition into a scleral plug for use in allowing the drug to be released into a vitreous body for treatment or prevention of intraocular diseases.

## Claims

1. A composition characterized by comprising a drug of protein derivatives adsorbed on a surface of fine particles of a lactic acid copolymer.

2. The composition according to claim 1, wherein an average diameter of the fine particles is less than 1,000 nanometers.

3. The composition according to claim 1, wherein an average diameter of the fine particles is within a range of 50 to 500 nanometers.

4. The composition according to claim 1, wherein the drug of protein derivatives is an interferon.

5. The composition according to claim 4, wherein the interferon is an interferon-β.

6. The composition according to claim 1, wherein the lactic acid copolymer comprises lactic acid units and glycolic acid units.

7. A composition characterized by comprising an interferon-β adsorbed on fine particles of a lactic acid copolymer that comprises lactic acid units and glycolic acid units, the fine particles having an average diameter of less than 1,000 nanometers.

8. A scleral plug formed from a composition characterized by comprising a drug of protein derivatives adsorbed on a surface of fine particles of a lactic acid copolymer.

9. The scleral plug according to claim 8, wherein an average diameter of the fine particles is less than 1,000 nanometers.

10. The scleral plug according to claim 8, wherein an average diameter of the fine particles is within a range of 50 to 500 nanometers.

11. The scleral plug according to claim 8, wherein the drug of protein derivatives is an interferon.

12. The scleral plug according to claim 11, wherein the interferon is an interferon-β.

13. The scleral plug according to claim 8, wherein the lactic acid copolymer comprises lactic acid units and glycolic acid units.

14. A scleral plug formed from a composition characterized by comprising an interferon-β adsorbed on fine particles of a lactic acid copolymer that comprises lactic acid units and glycolic acid units, the fine particles having an average diameter of less than 1,000 nanometers.

15. The scleral plug according to claim 8, wherein a weight average molecular weight of the lactic acid copolymer is within a range of 10,000 to 1,000,000.

16. The scleral plug according to claim 8, wherein a weight average molecular weight of the lactic acid copolymer is within a range of 20,000 to 200,000.

17. The scleral plug according to any one of claims 8 to 16, wherein a compositional molar ratio of the lactic acid units to the glycolic acid units is within a range of 50/50 to 100/0.

18. The scleral plug according to any one of claims 8 to 16, wherein a compositional molar ratio of the lactic acid units to the glycolic acid units is within a range of 50/50 to 80/20.

19. The scleral plug according to any one of claims 8 to 16, wherein a compositional molar ratio of the lactic acid units to the glycolic acid units is within a range of 70/30 to 80/20.

20. A scleral plug formed from a composition comprising a drug of protein derivatives adsorbed on a surface of fine particles of a lactic acid copolymer, the drug being gradually released into a vitreous body.

21. A scleral plug for treatment or prevention of an intraocular diseases, the scleral plug being formed from a composition characterized by comprising a drug of protein derivatives adsorbed on a surface of fine particles of a lactic acid copolymer, the drug being gradually released into a vitreous body.

22. The scleral plug according to claim 21, wherein the intraocular diseases is a retinal disease.

23. The scleral plug according to claim 22, wherein the retinal disease is diabetic retinopathy, macular degeneration, or retinal central vein obstruction.

24. A scleral plug formed from a composition characterized by comprising an interferon-β adsorbed on a surface of fine particles of a lactic acid copolymer, the lactic acid copolymer having a compositional molar ratio of lactic acid units to glycolic acid units within a range of 50/50 to 100/0 and having a weight average molecular weight of 10,000 to 1,000,000, the interferon-β being gradually released into a vitreous body.

25. A scleral plug formed from a composition characterized by being formed comprising an interferon-β adsorbed on a surface of fine particles of a lactic acid copolymer, the lactic acid copolymer having a compositional molar ratio of lactic acid units to glycolic acid units within a range of 70/30 to 80/20 and having a weight average molecular weight of 20,000 to 200,000, the interferon-β being gradually released into a vitreous body.

26. The scleral plug according to any one of claims 8 to 25, wherein the lactic acid units are in L-, D-, or DL-form.

27. The scleral plug according to any one of claims 8 to 26, manufactured by compression molding.
